(19) Europäisches Patentamt
European Patent Office
Office européen des brevets

(11) **EP 3 733 639 A1**

(12) **EUROPEAN PATENT APPLICATION**
published in accordance with Art. 153(4) EPC

(43) Date of publication:
**04.11.2020 Bulletin 2020/45**

(51) Int Cl.:
***C07C 67/29*** *(2006.01)*   ***B01J 21/10*** *(2006.01)*
***C07C 69/24*** *(2006.01)*   ***C07C 69/58*** *(2006.01)*
***C07B 61/00*** *(2006.01)*

(21) Application number: **18896969.5**

(22) Date of filing: **20.12.2018**

(86) International application number:
**PCT/JP2018/047012**

(87) International publication number:
**WO 2019/131442 (04.07.2019 Gazette 2019/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.12.2017 JP 2017250992**

(71) Applicant: **Lion Corporation**
**Tokyo 130-8644 (JP)**

(72) Inventors:
• **JOKO Akinori**
  **Tokyo 130-8644 (JP)**
• **SATO Masahiro**
  **Tokyo 130-8644 (JP)**
• **NIIKURA Fumiya**
  **Tokyo 130-8644 (JP)**

(74) Representative: **Cabinet Laurent & Charras**
**Le Contemporain**
**50 Chemin de la Bruyère**
**69574 Dardilly Cedex (FR)**

(54) **METHOD FOR PRODUCING FATTY ACID POLYOXYETHYLENE METHYL ETHER**

(57)    In a method for producing a fatty acid polyoxyethylene methyl ether according to the present invention, ethylene oxide is added to a fatty acid methyl ester component comprising, as a main component, a fatty acid methyl ester having a fatty acid residue of 18 to 22 carbon atoms, the addition being conducted in the presence of a composite metal oxide catalyst and at least one alcohol selected from the group consisting of diethylene glycol, ethylene glycol, propylene glycol, ethanol, methanol and isopropyl alcohol.

EP 3 733 639 A1

**Description**

TECHNICAL FIELD

The present invention relates to a method for producing a fatty acid polyoxyethylene methyl ether.

Priority is claimed on Japanese Patent Application No. 2017-250992, filed December 27, 2017, the content of which is incorporated herein by reference.

BACKGROUND ART

Fatty acid polyoxyethylene methyl ethers are used as nonionic surfactants in a variety of fields.

One known method for producing fatty acid polyoxyethylene methyl ethers involves adding ethylene oxide to a fatty acid methyl ester in the presence of a composite metal oxide catalyst. The solid composite metal oxide catalyst is typically removed from the product by a solid-liquid separation following the reaction.

In order to make this solid-liquid separation following the reaction unnecessary, a method has been proposed in which, during production of the alkylene oxide adduct, a polyhydric alcohol such as glycerol is added together with the composite metal oxide catalyst (Patent Document 1).

PRIOR ART LITERATURE

Patent Document

Patent Document 1: International Patent Publication WO 2007/113985

DISCLOSURE OF INVENTION

Problems to be Solved by the Invention

When ethylene oxide is added to a fatty acid methyl ester in the presence of a composite metal oxide catalyst, a problem arises in that there is an induction period in the initial stage of the reaction, meaning the reaction time lengthens. The induction period develops as a result of the time required for the catalytic action to manifest, which leads to a lengthening of the reaction process time.

Patent Document 1 discloses that as the amount used of the polyhydric alcohol is increased, a longer time is required for the catalytic action to manifest in the initial stage of the reaction, namely the induction period lengthens.

The present invention has an object of providing a method for producing a fatty acid polyoxyethylene methyl ether which enables a reduction in the induction period when adding ethylene oxide to a fatty acid methyl ester in the presence of a composite metal oxide catalyst.

Means for Solving the Problems

The present invention has the aspects described below.

[1] A method for producing a fatty acid polyoxyethylene methyl ether, the method including adding ethylene oxide to a fatty acid methyl ester component containing, as the main component, a fatty acid methyl ester having a fatty acid residue of 18 to 22 carbon atoms, wherein the addition is conducted in the presence of a composite metal oxide catalyst and at least one alcohol selected from the group consisting of diethylene glycol, ethylene glycol, propylene glycol, ethanol, methanol and isopropyl alcohol.

[2] The method for producing a fatty acid polyoxyethylene methyl ether according to [1], wherein at least a portion of the fatty acid methyl ester having a fatty acid residue of 18 to 22 carbon atoms has an unsaturated fatty acid residue.

[3] The method for producing a fatty acid polyoxyethylene methyl ether according to [1] or [2], wherein the fatty acid methyl ester having a fatty acid residue of 18 to 22 carbon atoms contains a fatty acid methyl ester having a fatty acid residue of 18 carbon atoms.

[4] The method for producing a fatty acid polyoxyethylene methyl ether according to any one of [1] to [3], wherein the fatty acid methyl ester component is a fatty acid methyl ester mixture derived from a distillation fraction of 18 carbon atoms from palm oil, palm kernel oil, coconut oil or soybean oil, a fatty acid methyl ester mixture derived from a distillation fraction of 18 to 22 carbon atoms from rapeseed oil, or a mixture of two or more such mixtures.

[5] The method for producing a fatty acid polyoxyethylene methyl ether according to any one of [1] to [4], wherein the amount of the alcohol is within a range from 0.06 to 0.25% by mass relative to the total mass of the fatty acid methyl ester component and the ethylene oxide.

[6] The method for producing a fatty acid polyoxyethylene methyl ether according to any one of [1] to [5], wherein the amount of the composite metal oxide catalyst is within a range from 0.1 to 0.2% by mass relative to the total mass of the fatty acid methyl ester component and the ethylene oxide.

[7] The method for producing a fatty acid polyoxyethylene methyl ether according to any one of [1] to [6], wherein the composite metal oxide catalyst has undergone surface modification with a metal hydroxide.

[8] The method for producing a fatty acid polyoxyethylene methyl ether according to [7], wherein the amount of the metal hydroxide is within a range from 2 to 4% by mass relative to the mass of the composite metal oxide catalyst.

Effects of the Invention

The present invention is able to provide a method for producing a fatty acid polyoxyethylene methyl ether that enables a reduction in the induction period when adding ethylene oxide to a fatty acid methyl ester in the presence of a composite metal oxide catalyst.

EMBODIMENTS FOR CARRYING OUT THE INVENTION

In this description and in the claims, expressions such as "a to b" denoting numerical ranges are deemed to include the numerical values of a and b as the lower limit and upper limit respectively.

The method for producing a fatty acid polyoxyethylene methyl ether according to the present invention has a step of adding ethylene oxide to a fatty acid methyl ester component in the presence of an alcohol and a composite metal oxide catalyst (the addition step).

After the addition step, a step of purifying the product produced in the addition step may be conducted if necessary (the purification step).

(Alcohol)

The alcohol is used to reduce the induction period.

The alcohol is at least one compound selected from the group consisting of diethylene glycol, ethylene glycol, propylene glycol, ethanol, methanol and isopropyl alcohol. One of these alcohols may be used alone, or a combination of two or more alcohols may be used.

Diethylene glycol, ethylene glycol and propylene glycol are preferred as the alcohol, and in terms of achieving a superior reduction effect on the induction period, diethylene glycol is more preferred.

(Composite Metal Oxide Catalyst)

Examples of the composite metal oxide catalyst include magnesium oxide to which one or metal ions such as $Al^{3+}$, $Ga^{3+}$, $In^{3+}$, $Tl^{3+}$, $Co^{3+}$, $Sc^{3+}$, $La^{3+}$ or $Mn^{2+}$ have been added (for example, catalysts disclosed in Japanese Examined Patent Application, Second Publication No. Hei 6-15038, Japanese Unexamined Patent Application, First Publication No. Hei 7-227504, Japanese Unexamined Patent Application, First Publication No. Hei 6-198169, Japanese Unexamined Patent Application, First Publication No. Hei 6-182206, and Japanese Unexamined Patent Application, First Publication No. Hei 5-170688); and aluminum-magnesium-based composite metal oxide catalysts such as calcined hydrotalcite (for example, the catalysts disclosed in Japanese Unexamined Patent Application, First Publication No. Hei 2-71841) and calcined products of aluminum magnesium hydroxide (for example, the catalysts disclosed in Japanese Unexamined Patent Application, First Publication No. Hei 8-268919). One of these composite metal oxide catalysts may be used alone, or a combination of two or more such catalysts may be used.

The composite metal oxide catalyst is typically in particulate form. In the following description, the composite metal oxide catalyst in particulate form is also referred to using the term "catalyst particles".

Specific examples of the calcined products of aluminum magnesium hydroxide include compounds represented by formula (1) shown below. A calcined product of aluminum magnesium hydroxide can be obtained by calcining a coprecipitate of aluminum hydroxide and magnesium hydroxide.

$$nMgO \cdot Al_2O_3 \cdot mH_2O \qquad (1)$$

In formula (1), n and m each represent a positive number.

Further, n is preferably within a range from 1 to 3, and is particularly preferably about 2.5.

There are no particular limitations on m, which may be selected appropriately in accordance with the desired purpose.

Among the various calcination conditions used when obtaining a calcined hydrotalcite or a calcined product of aluminum magnesium hydroxide, the calcination temperature may be selected in accordance with the intended purpose, but in terms of ensuring favorable catalytic action manifestation and suppressing the production of by-products, the calcination temperature is preferably within a range from 400 to 950°C, and more preferably from 800 to 900°C. The calcination time may be selected as appropriate, but when the calcination is performed at a temperature within the above range, is typically from 2 to 4 hours.

If the calcination temperature is too low, then a problem of reduced catalytic activity and a problem of increased production of by-products tend to occur. On the other hand, if the calcination temperature is too high, a problem of reduced catalytic activity tends to occur. In other words, the catalyst calcination conditions are preferably controlled appropriately from the two viewpoints of maintaining high catalytic activity and reducing the amount of by-products.

During the calcination, the specific surface area of the catalyst particles may be used as a process control indicator.

The specific surface area of the catalyst particles may be, for example, within a range from 100 to 200 $m^2$/g.

The specific surface area of the catalyst particles describes a value measured using a BET specific surface area measurement device (such as the specific surface area measurement device SA-1000 manufactured by Sibata Scientific Technology Ltd.).

There are no particular limitations on the average particle size of the catalyst particles, which may be, for example, within a range from 10 to 1,000 $\mu$m.

The average particle size of the catalyst particles describes a median value measured using a laser diffraction/scattering particle size distribution analyzer (LA-920 manufactured by Horiba, Ltd.), using acetonitrile as the dispersion medium.

During the ethylene oxide addition reaction, for example, the catalyst particles can disintegrate due to accumulation of the heat of reaction inside the catalyst particles, the production of high-molecular weight by-products, the occurrence of sudden changes in pressure, and mechanical shearing caused by a stirring blade or the like, and therefore the average particle size of the catalyst can vary. Moreover, polyhydric alcohols can sometimes also cause further micronization of the catalyst particles. Accordingly, the average particle size of the catalyst contained in the product following reaction may be, for example, within a range from 0.1 to 500 $\mu$m.

The composite metal oxide catalyst has preferably undergone surface modification with a metal hydroxide. When reaction is conducted using a composite metal oxide catalyst that has undergone surface modification with a metal hydroxide, the amount of residual unreacted fatty acid methyl ester tends to be small, and a fatty acid polyoxyethylene methyl ether having a narrow distribution for the number of added moles of ethylene oxide (a high narrow ratio) can be obtained. The metal hydroxide selectively partially poisons (inactivates) active acid sites which exist in high density on the surface of the composite metal oxide catalyst.

The metal hydroxide is preferably a hydroxide of an alkali metal or an alkaline earth metal, and is more preferably sodium hydroxide or potassium hydroxide.

The surface modification of the composite metal oxide catalyst with a metal hydroxide can be conducted, for example, using the method disclosed in Japanese Unexamined Patent Application, First Publication No. Hei 8-169860 or Japanese Unexamined Patent Application, First Publication No. Hei 8-169861.

The amount of the metal hydroxide used in the surface modification may be selected appropriately in accordance with the intended purpose, but the amount of the metal hydroxide relative to the mass of the composite metal oxide catalyst (the unmodified composite metal oxide catalyst) is, for example, preferably within a range from 1 to 4% by mass, more preferably from 2 to 4% by mass, and particularly preferably from 2 to 3% by mass. Provided the amount of the metal hydroxide is at least as large as the above lower limit, the effects of the surface modification can be adequately realized. Further, provided the amount of the metal hydroxide is not more than the above upper limit, the performance of the composite metal oxide catalyst remains favorable, and the reaction time of the addition reaction can be shortened.

(Fatty Acid Methyl Ester Component)

The fatty acid methyl ester component is a component containing at least one fatty acid methyl ester, and contains a fatty acid methyl ester having a fatty acid residue of 18 to 22 carbon atoms (hereafter also referred to as a "C18 to C22 fatty acid methyl ester") as the main component. Using a C18 to C22 fatty acid methyl ester as the main component enables the reduction effect on the induction period provided by the alcohol to be achieved.

The fatty acid methyl ester component may be composed solely of the C18 to C22 fatty acid methyl ester, or may be a mixture of the C18 to C22 fatty acid methyl ester and another fatty acid methyl ester. This other fatty acid methyl ester is preferably a fatty acid methyl ester having a fatty acid residue of 10 to 17 carbon atoms. In other words, the fatty acid methyl esters that constitute the fatty acid methyl ester component are preferably fatty acid methyl esters having a fatty acid residue of 10 to 22 carbon atoms.

A "fatty acid residue" means a group obtained by removing the OH from the carboxyl group of the fatty acid.

The term "main component" means that the proportion of the C18 to C22 fatty acid methyl ester, relative to the total mass of the fatty acid methyl ester component, is at least 50% by mass.

The proportion of the C18 to C22 fatty acid methyl ester, relative to the total mass of the fatty acid methyl ester component, is preferably at least 85% by mass, and more preferably 95% by mass or greater. The upper limit for this proportion is not particularly limited, and may be 100% by mass.

The fatty acid methyl ester component is composed of one or more fatty acid methyl esters represented by formula (2) shown below, wherein a C18 to C22 fatty acid methyl ester in which $R^1$ in formula (2) represents a saturated or unsaturated hydrocarbon group of 17 to 21 carbon atoms is preferably the main component. This fatty acid methyl ester component may or may not contain a fatty acid methyl ester in which $R^1$ in formula (2) represents a saturated or unsaturated hydrocarbon group of 9 to 16 carbon atoms.

$$R^1COOCH_3 \qquad (2)$$

In formula (2), $R^1$ represents a saturated or unsaturated hydrocarbon group of 9 to 21 carbon atoms. Accordingly, the fatty acid residue ($R^1CO$) of the fatty acid methyl ester represented by formula (2) has 10 to 22 carbon atoms.

The saturated or unsaturated hydrocarbon group is preferably linear or branched.

An unsaturated hydrocarbon group contains an unsaturated bond such as a double bond or triple bond between two carbon atoms. The number of unsaturated bonds in the unsaturated hydrocarbon group is, for example, from 1 to 3.

The C18 to C22 fatty acid methyl ester in the fatty acid methyl ester component may be one compound or two or more compounds.

At least a portion of the C18 to C22 fatty acid methyl ester preferably has an unsaturated fatty acid residue (a $R^1CO$ group in which $R^1$ is an unsaturated hydrocarbon group of 17 to 21 carbon atoms). In other words, the fatty acid methyl ester component preferably contains a fatty acid methyl ester having an unsaturated fatty acid residue of 18 to 22 carbon atoms. This ensures a superior reduction effect on the induction period.

The unsaturated fatty acid residue of 18 to 22 carbon atoms typically contains 1 to 3 double bonds.

The proportion of the C18 to C22 fatty acid methyl ester having an unsaturated fatty acid residue, relative to the total mass of the C18 to C22 fatty acid methyl ester (hereafter also referred to as the "C18 to C22 unsaturated fatty acid residue ratio") is preferably at least 70% by mass, more preferably at least 80% by mass, and even more preferably 88% by mass or greater. Provided the C18 to C22 unsaturated fatty acid residue ratio is at least as high as the above lower limit, the reduction effect on the induction period is superior. Although there are no particular limitations on the upper limit for the C18 to C22 unsaturated fatty acid residue ratio, in terms of ease of production and availability, a ratio of 95% by mass or less is preferred.

The C18 to C22 unsaturated fatty acid residue ratio may be, for example, within a range from 70 to 95% by mass, from 80 to 95% by mass, or from 88 to 95% by mass.

The C18 to C22 unsaturated fatty acid residue ratio may employ a known value, or may be a value measured by a conventional method such as gas chromatography using an HP-INNOWax column manufactured by Agilent Technologies, Inc..

The C18 to C22 fatty acid methyl ester preferably contains a fatty acid methyl ester having a fatty acid residue of 18 carbon atoms (hereafter also referred to as a "C18 fatty acid methyl ester").

The amount of the C18 fatty acid methyl ester, relative to the total mass of the C18 to C22 fatty acid methyl ester, is preferably at least 50% by mass, more preferably at least 90% by mass, and even more preferably 99% by mass or greater. The upper limit for this amount is not particularly limited, and may be 100% by mass.

Further, at least a portion of the C18 fatty acid methyl ester preferably has an unsaturated fatty acid residue. The proportion of the C18 fatty acid methyl ester having an unsaturated fatty acid residue, relative to the total mass of the C18 fatty acid methyl ester (hereafter also referred to as the "C18 unsaturated fatty acid residue ratio") is preferably at least 70% by mass, more preferably at least 80% by mass, and even more preferably 88% by mass or greater. The upper limit for this proportion is not particularly limited, and may be 100% by mass.

Examples of materials that may be used as the fatty acid methyl ester component include fatty acid methyl ester mixtures derived from a distillation fraction of 18 carbon atoms from palm oil, palm kernel oil, coconut oil or soybean oil, fatty acid methyl ester mixtures derived from a distillation fraction of 18 to 22 carbon atoms from rapeseed oil, or a mixture of two or more such mixtures.

Among the above, in terms of containing low numbers of linoleic acid residues and linolenic acid residues and exhibiting superior oxidation resistance, a fatty acid methyl ester mixture derived from a distillation fraction of 18 carbon atoms from palm oil, palm kernel oil or coconut oil, or a mixture of two or more such mixtures is preferred, and in terms of containing a high proportion of the distillation fraction of 18 carbon atoms and ease of availability, a fatty acid methyl ester mixture derived from a distillation fraction of 18 carbon atoms from palm oil is particularly preferred.

In those cases where a fatty acid methyl ester having a longer chain length and a high unsaturated fatty acid residue ratio is used, a fatty acid methyl ester mixture derived from a distillation fraction of 18 to 22 carbon atoms from rapeseed oil may be used.

These fatty acid methyl ester mixtures may employ mixtures obtained using conventional production methods, or may be commercially available products.

(Addition Step)

In the addition step, ethylene oxide is added to the fatty acid methyl ester component in the presence of the alcohol and composite metal oxide catalyst described above.

The amount of the alcohol used in the addition step, relative to the total mass of the fatty acid methyl ester component and the ethylene oxide, is preferably within a range from 0.06 to 0.25% by mass, and more preferably from 0.10 to 0.20% by mass. Provide the amount of the alcohol is at least as large as the above lower limit, a reduction effect on the induction period can be more easily obtained. Provided the amount of the alcohol is not more than the above upper limit, the purification time can be shortened in those cases where a purification step is conducted after the addition step.

The amount of the composite metal oxide catalyst used in the addition step, relative to the total mass of the fatty acid methyl ester component and the ethylene oxide, is preferably within a range from 0.1 to 0.2% by mass, and more preferably from 0.10 to 0.15% by mass. Provided the amount of the composite metal oxide catalyst is at least as large as the above lower limit, the reaction rate is fast, and the reaction time can be shortened. Further, provided the amount of the composite metal oxide catalyst is not more than the above upper limit, the purification time can be shortened in those cases where a purification step is conducted after the addition step. In those cases where the composite metal oxide catalyst has undergone surface modification with a metal hydroxide, the amount of the composite metal oxide catalyst represents the amount of the catalyst in an unmodified state.

The amount of ethylene oxide used in the addition step may be selected appropriately in accordance with factors such as the type of fatty acid methyl ester that is used, and the performance required of the targeted fatty acid polyoxyethylene methyl ether, but the amount of ethylene oxide per 1 mol of the fatty acid methyl ester is preferably within a range from 1 to 50 mol, more preferably from 3 to 30 mol, and particularly preferably from 5 to 20 mol.

In the addition step, it is preferable that the composite metal oxide catalyst and the alcohol are first brought into contact, and the ethylene oxide is then added to the fatty acid methyl ester component in the presence of the catalyst and alcohol.

In order to bring the composite metal oxide catalyst and the alcohol into contact, the composite metal oxide catalyst and the alcohol may be mixed. At this time, the fatty acid methyl ester component may be mixed together with the catalyst and alcohol, and if necessary, may be mixed together with the metal hydroxide used for surface modification of the composite metal oxide catalyst.

In one preferred aspect of the addition step, first, the fatty acid methyl ester component, the composite metal oxide catalyst, the alcohol, and if necessary the metal hydroxide, are introduced into a reactor in amounts that satisfy the respective preferred ranges described above, the atmosphere inside the reactor is replaced with nitrogen while the reactor contents are stirred and mixed, and a dehydration is then conducted under heating and reduced pressure conditions. Subsequently, the temperature and pressure inside the reactor are adjusted, and ethylene oxide is introduced (supplied) at a prescribed rate in an amount that falls within the preferred range described above. As a result, the ethylene oxide addition reaction proceeds, and a fatty acid polyoxyethylene methyl ether is produced. Following introduction of the ethylene oxide, an aging reaction may be conducted if necessary.

There are no particular limitations on the reactor, which may be selected appropriately in accordance with the intended purpose. Examples include typical stirred tank batch reactors such as autoclaves and the like.

The method used for introducing the various components into the reactor is not particularly limited, and may be selected appropriately in accordance with the intended purpose. In terms of the composite metal oxide catalyst, a method in which the catalyst is introduced in the form of a powder by suction into the reactor under a state of reduced pressure is preferred from the viewpoint that the resulting pressure difference further accelerates micronization of the catalyst particles.

The temperature during substitution of the atmosphere inside the reactor with nitrogen may be selected appropriately in accordance with the intended purpose, but is preferably within a range from 0 to 90°C, and more preferably from 20 to 70°C. Provided this temperature is at least as high as the above lower limit, the state of fluidity of the added raw materials is favorable, the composite metal oxide catalyst and the alcohol may satisfactory contact, and the effect of the alcohol is more readily obtained. Provided the temperature is not higher than the above upper limit, the moisture contained in the added raw materials is less likely to undergo interactions with the surface of the composite metal oxide catalyst, meaning the desired catalytic performance can be more easily achieved.

The pressure during substitution of the atmosphere inside the reactor with nitrogen is not particularly limited, and may be selected appropriately in accordance with the intended purpose.

The temperature during the dehydration conducted under heating and reduced pressure conditions may be selected appropriately in accordance with the intended purpose, but is preferably within a range from 70 to 150°C, and more preferably from 90 to 130°C. Provided this temperature is at least as high as the above lower limit, a satisfactory dehydration effect can be obtained, and problems such as the residual moisture causing the production of by-products or adversely affecting the reactivity of the catalyst can be suppressed. Provided the temperature is not higher than the above upper limit, distillation and discharge of the added raw materials from the reaction system can be suppressed.

The pressure during the dehydration conducted under heating and reduced pressure conditions may be selected appropriately in accordance with the intended purpose, but is preferably not more than 13 kPa, and more preferably 4 kPa or less. Provided this pressure is not higher than the above upper limit, a satisfactory dehydration effect is obtained, and problems such as residual moisture causing the production of by-products or adversely affecting the reactivity of the catalyst can be suppressed. The lower limit for the pressure is not particularly limited, and for example, may be 4 kPa.

The reaction temperature during introduction (supply) of the ethylene oxide into the reactor to perform the addition reaction may be set appropriately in accordance with the activity and the like of the catalyst that is used, but is preferably within a range from 120 to 230°C, and more preferably from 150 to 200°C. Provided this temperature is at least as high as the above lower limit, the catalytic activity is satisfactorily high, and the reaction time can be shortened. Provided the temperature is not higher than the above upper limit, decomposition of the reaction raw materials or the product is less likely to occur.

The reaction pressure during introduction (supply) of the ethylene oxide into the reactor to perform the addition reaction may be set appropriately in accordance with the reaction temperature and the activity and the like of the catalyst that is used, or in terms of the upper limit pressure, may be set appropriately to a value within a conventionally used range, in accordance with the designed pressure resistance of the reactor being used. For example, the upper limit pressure is preferably within a range from 0.1 to 2.0 MPa, and more preferably from 0.2 to 1.0 MPa.

The ethylene oxide introduction rate (supply rate) into the reactor during introduction (supply) of the ethylene oxide into the reactor to perform the addition reaction may be set appropriately in accordance with the intended purpose, but the ethylene oxide supply rate Va is preferably controlled so that the value of F (/min) represented by formula (3) shown below satisfies a range from 0.01 to 0.07.

$$F = Va/Mp \qquad (3)$$

In formula (3), Va is the supply rate (mol/min) of ethylene oxide provided to the reaction, and Mp is the number of moles (mol) of fatty acid polyoxyethylene methyl ether produced by the reaction.

Provided the value of F is at least as high as the above lower limit, the reaction time can be satisfactorily shortened. Provided the value of F is not more than the above upper limit, the supply rate of ethylene oxide is satisfactorily slow relative to the catalytic activity, and the pressure inside the reactor can be prevented from exceeding the preferred upper limit. Further, the production of by-product high-molecular weight polyethylene glycols can be suppressed.

The reaction time for the addition step is, for example, from 3 to 8 hours.

The reaction time is the period from the completion of the induction period until the completion of the introduction of all of the ethylene oxide. The induction period is the period from the start of introduction of ethylene oxide into the reactor until a reduction in the pressure inside the reactor is observed.

The product from the addition reaction includes not only the fatty acid polyoxyethylene methyl ether, but also the composite metal oxide catalyst and the alcohol and the like. Further, the product sometimes also includes unreacted fatty acid methyl ester and polyethylene glycol by-products.

In those cases where the composite metal oxide catalyst is included in the product at a size (for example, an average particle size exceeding 2 μm) that requires the catalyst to be treated as substantially particles, the catalyst is preferably removed in the purification step described below. In those cases where the composite metal oxide catalyst has been micronized to a size (for example, an average particle size of 2 μm or less) that does not require the catalyst to be treated as substantially particles, the catalyst need not be removed in the purification step.

(Purification Step)

In the purification step, the product obtained in the addition step is purified.

For example, in the purification step the composite metal oxide catalyst and any polyethylene glycol within the product are removed.

The purification of the product may be conducted using conventional methods. For example, water, and if necessary citric acid or malic acid or the like, may be added to the product to aggregate polyethylene glycol by-product, and a solid-liquid separation such as filtration or centrifugal separation may then be used to remove the composite metal oxide catalyst and the polyethylene glycol and the like.

By conducting the method described above, a fatty acid polyoxyethylene methyl ether is obtained. The average number of added moles of ethylene oxide in the fatty acid polyoxyethylene methyl ether is typically the same as the number of moles of ethylene oxide used per 1 mol of the fatty acid methyl ester in the addition step.

The fatty acid polyoxyethylene methyl ether that is obtained corresponds with the fatty acid residue of the raw material fatty acid methyl ester component. Because the fatty acid methyl ester component contains a C18 to C22 fatty acid methyl ester as the main component, the fatty acid polyoxyethylene methyl ether contains a fatty acid polyoxyethylene methyl ether having a fatty acid residue of 18 to 22 carbon atoms as the main component. Further, the ratio of the number of moles of the fatty acid polyoxyethylene methyl ether having a fatty acid residue of 18 to 22 carbon atoms relative to the total number of moles of fatty acid polyoxyethylene methyl ether is the same as the ratio of the number of moles of the C18 to C22 fatty acid methyl ester relative to the total number of moles of the fatty acid methyl ester component.

For example, when the fatty acid methyl ester component is composed of fatty acid methyl esters represented by formula (2) shown above, and contains a C18 to C22 fatty acid methyl ester in which $R^1$ in formula (2) is a saturated or unsaturated hydrocarbon group of 17 to 21 carbon atoms as the main component, a fatty acid polyoxyethylene methyl ether is obtained that is composed of compounds represented by formula (4) shown below, and contains a compound in which $R^1$ in formula (4) is a saturated or unsaturated hydrocarbon group of 17 to 21 carbon atoms as the main component. When the fatty acid methyl ester component also contains a fatty acid methyl ester in which $R^1$ in formula (2) is a saturated or unsaturated hydrocarbon group of 9 to 16 carbon atoms, the obtained fatty acid polyoxyethylene methyl ether will also contain a compound in which $R^1$ in formula (4) is a saturated or unsaturated hydrocarbon group of 9 to 16 carbon atoms.

$$R^1CO(OE)_pOCH_3 \qquad (4)$$

In formula (4), $R^1$ and $R^2$ are the same as defined above, E represents an ethylene group, and p is a positive integer.

$(OE)_p$ is formed by the addition of ethylene oxide. Further, p corresponds with the average number of added moles of ethylene oxide.

In the fatty acid polyoxyethylene methyl ether, the average number of added moles of ethylene oxide per 1 mol of the fatty acid methyl ester is preferably within a range from 1 to 50, more preferably from 3 to 30, and even more preferably from 5 to 20.

In those cases where the fatty acid polyoxyethylene methyl ether is used as a nonionic surfactant, the fatty acid polyoxyethylene methyl ether preferably has a structure that yields an HLB value (Griffin's method) of 3 to 20.

In the fatty acid polyoxyethylene methyl ether, the narrow ratio, which indicates the distribution ratio of compounds (ethylene oxide adducts) having different numbers of added moles of ethylene oxide, is preferably within a range from 30 to 75% by mass, and more preferably from 45 to 60% by mass. A higher narrow ratio, namely a narrower distribution, yields superior solubility at low temperature. Further, the higher the narrow ratio becomes, the smaller the amounts of raw material fatty acid methyl ester and ethylene oxide adducts having a small number (for example, 1 to 2) of added moles of ethylene oxide, resulting in reduced odor.

The narrow ratio can be determined using the method described below in the examples. The narrow ratio can be controlled, for example, by adjusting the amount of the metal hydroxide used in the surface modification of the composite metal oxide catalyst.

There are no particular limitations on the applications for the fatty acid polyoxyethylene methyl ether, and for example, the fatty acid polyoxyethylene methyl ether can be used as a surfactant, detergent, emulsifier, dispersant, oil-phase component modifier, penetrant, recycled paper deinking agent, or agricultural spreading agent or the like, in domestic, industrial and agricultural fields and the like.

In the production method of the present invention described above, because a specific alcohol and a fatty acid methyl ester component containing a C18 to C22 fatty acid methyl ester as the main component are combined, the induction period in the initial stage of the reaction can be reduced. For example, the induction period measured using the method described below in the examples can be reduced to less than 0.5 hours, and even to less than 0.25 hours. Because the induction period can be reduced, the production time for the fatty acid polyoxyethylene methyl ether can be shortened.

Furthermore, in those cases where the composite metal oxide catalyst is subjected to surface modification with a metal hydroxide to increase the narrow ratio of the fatty acid polyoxyethylene methyl ether, using the alcohol described above means that, compared with the case where the alcohol is not added or cases where glycerol is added, a high narrow ratio can be achieved even when the amount used of the metal hydroxide is reduced. By reducing the amount of the metal hydroxide, any reduction in the reaction rate of the addition reaction or resulting lengthening of the reaction time can be suppressed.

Conventionally, when ethylene oxide is added to a fatty acid methyl ester component in the presence of a composite metal oxide catalyst, the reaction period included an induction period. Specifically, an amount of time was required from the start of introduction of ethylene oxide into the reactor until manifestation of the action of the composite metal oxide catalyst, during which the pressure inside the reactor did not decrease (indicating that the ethylene oxide reaction was not proceeding).

In those cases where glycerol is used instead of the alcohol described above, the induction period lengthens even if the fatty acid methyl ester component has a C18 to C22 fatty acid methyl ester as the main component. Even when the alcohol described above is used, when a fatty acid methyl ester component containing a fatty acid methyl ester with a fatty acid residue of 12 carbon atoms as the main component is used instead of the fatty acid methyl ester component described above, the induction period lengthens.

EXAMPLES

The present invention is described below in further detail using a series of examples, but the present invention is in no way limited by these examples. In these examples, unless specifically stated otherwise, the terms "%" and "parts" refer to "% by mass" and "parts by mass" respectively.

The raw materials used in the examples are as described below.

<Raw Materials Used>

PASTELL M182 (product name): manufactured by Lion Specialty Chemicals Co., Ltd. (a fatty acid methyl ester mixture derived from a distillation fraction of 18 carbon atoms from palm oil. C16/C18:0/C18:1/C18:2 = 3/10/70/17 (mass ratio)). The numerical value following "C" indicates the number of carbon atoms in the fatty acid residue. The value of "X" in "C18:X" indicates the number of double bonds in the fatty acid residue.

PASTELL M12 (product name): manufactured by Lion Specialty Chemicals Co., Ltd. (a fatty acid methyl ester derived from a distillation fraction of 12 carbon atoms from palm oil).

Composite metal oxide catalyst: aluminum magnesium hydroxide calcined product obtained in Production Example 1 described below.

Glycerol: manufactured by Kanto Chemical Co., Inc.

Diethylene glycol: manufactured by Kanto Chemical Co., Inc.

KOH: manufactured by Kanto Chemical Co., Inc.

Citric acid monohydrate: manufactured by Kanto Chemical Co., Inc.

KC Flock W-50S (product name): manufactured by Nippon Paper Industries Co., Ltd.

Hyflow Supercel (product name): manufactured by Wako Pure Chemical Industries, Ltd.

<Production Example 1: Preparation of Composite Metal Oxide Catalyst>

Aluminum magnesium hydroxide with a chemical composition of $2.5MgO \cdot Al_2O_3 \cdot mH_2O$ (KW-300, manufactured by Kyowa Chemical Industry Co., Ltd.) was calcined at 900°C for 3 hours to obtain a magnesium-aluminum composite metal oxide catalyst powder.

<Example 1>

(Addition of Ethylene Oxide)

A 4 L autoclave was charged with 1,005 g of PASTELL M182, 2.5 g of the composite metal oxide catalyst, 3.13 g of diethylene glycol, and 0.1875 g of a 40% KOH aqueous solution (an equivalent mass of KOH of 3% relative to the composite metal oxide catalyst), the atmosphere inside the autoclave was substituted with nitrogen while the contents were stirred and mixed at 25°C, the temperature was then increased, and a dehydration was conducted at 100°C under reduced pressure (1.33 kPa) for 30 minutes. Subsequently, at a temperature of 180°C and with the upper limit for the reaction pressure set to 0.6 MPa, 1,489 g of ethylene oxide (a 10-fold molar equivalence relative to the PASTELL M182) was introduced. An aging reaction was conducted by continuing stirring for a further 30 minutes, and the reaction mixture was then cooled to room temperature (25°C), thus obtaining a product containing a fatty acid polyoxyethylene methyl ether (MEE) in which the average number of added moles of ethylene oxide was 10. The obtained product was extracted from the autoclave.

(Product Purification)

A pressure vessel fitted with a stirrer and a temperature adjustment device was charged with 1,200 g of the above product, and the product was heated to 80°C. Subsequently, 63 g of ion-exchanged water was added to form a water dilution. A pH adjustment was performed by adding citric acid monohydrate to the water dilution to adjust the pH (the pH of a dilution obtained by diluting a sample of the water dilution with distilled water to obtain an ethylene oxide adduct concentration of 5%) to a value of 7.6, and the resulting mixture was stirred for 15 minutes with the temperature held at 80°C. Subsequently, 3.88 g of Hyflow Supercel (0.3% relative to the water dilution) and 6.31 g of KC Flock W-50S (0.5% relative to the water dilution) were added as filtration aids, and the resulting mixture was stirred for 15 minutes. Subsequently, 200 g of the water dilution containing the filtration aids was extracted, 0.25 g of Hyflow Supercel (0.2 $kg/m^2$ relative to the filtration surface area) and 1.25 g of KC Flock W-50S (1.0 $kg/m^2$ relative to the filtration surface area) were added as precoating agents, and following uniform dispersion, precoating of the filtration material (a metal mesh filter) was conducted. Using the precoated filtration material, the remaining water dilution was subjected to a main filtration, thereby removing the composite metal oxide catalyst by filtration and obtaining a purified product.

(Measurement of Narrow Ratio)

Using high-performance liquid chromatography (HPLC) under the measurement conditions described below, the distribution in the obtained purified product of ethylene oxide adducts having different numbers of added moles of ethylene oxide was measured, and the MEE narrow ratio was calculated using formula (5) shown below. The result is shown in Table 1.

[Conditions for Measuring Distribution of Ethylene Oxide Adducts by HPLC]

Apparatus: LC-6A (manufactured by Shimadzu Corporation)
Detector: SPD-10A
Measurement wavelength: 220 nm
Column: Zorbax C8 (manufactured by DuPont Corporation)
Mobile phase: acetonitrile/water = 60/40 (volumetric ratio)
Flow rate: 1 mL/min
Temperature: 20°C

[Mathematical Formula 1]

$$\text{Narrow ratio} = \sum_{i=n_{\max}-2}^{i=n_{\max}+2} Yi \qquad \text{Formula (5)}$$

In formula (5), $n_{\max}$ represents the number of added moles of ethylene oxide in the ethylene oxide adduct that exists in the largest amount among all of the ethylene oxide adducts.
Further, i represents the number of added moles of ethylene oxide.
Yi represents the proportion (% by mass) of the ethylene oxide adduct in which the number of added moles of ethylene oxide is i relative to all of the ethylene oxide adducts.

(Induction Period, Reaction Time, Purification Time)

The induction period and the reaction time in the ethylene oxide addition described above were measured using the criteria described below. Further, the purification time during the purification of the product was measured using the criterion described below. The results are shown in Table 1.
Induction period: the period from the start of ethylene oxide introduction into the autoclave until a decrease in the pressure inside the autoclave is observed.
Reaction time: the time from the end of the induction period until completion of introduction of all of the ethylene oxide.
Purification time: the period from the start of the main filtration of the water dilution until completion of filtration of all of the water dilution.

<Examples 2 to 10, Comparative Examples 1 to 5>

With the exceptions of using the types and amounts of alcohol, the types of fatty acid methyl ester component, the amounts of the composite metal oxide catalyst (hereafter also referred to as the "catalyst amount") and the amounts of the 40% KOH aqueous solution (hereafter also referred to as the "alkali amount") shown in Tables 1 to 3, the same operations as those described for Example 1 were performed, and the narrow ratio, the induction period, the reaction time and the purification time were measured. The results are shown in Tables 1 to 3.

In Tables 1 to 3, "C18" indicates PASTELL M182, and "C12" indicates PASTELL M12. The alcohol amount and catalyst amount represents proportions relative to the total mass of the fatty acid methyl ester component and the ethylene oxide. The alkali amount represents the amount of alkali metal hydroxide (KOH) relative to the amount of the composite metal oxide catalyst.

[Table 1]

| | | Example 1 | Example 2 | Example 3 | Example 4 | Example 5 |
|---|---|---|---|---|---|---|
| Conditions | Fatty acid methyl ester component | C18 | C18 | C18 | C18 | C18 |
| | Alcohol | DEG | DEG | DEG | DEG | DEG |
| | Alcohol amount (%) | 0.125 | 0.06 | 0.25 | 0.125 | 0.125 |
| | Catalyst amount (%) | 0.1 | 0.1 | 0.1 | 0.1 | 0.1 |
| | Alkali amount (% relative to catalyst) | 3 | 3 | 3 | 2 | 4 |
| Evaluations | Narrow ratio (%) | 55 | 55 | 55 | 46 | 73 |
| | Induction period (h) | 0 | 0 | 0 | 0 | 0 |
| | Reaction time (h) | 4.5 | 4.5 | 4.5 | 4 | 5 |
| | Purification time (h) | 5 | 4.5 | 5.5 | 5 | 5 |

[Table 2]

| | | Example 6 | Comparative Example 1 | Comparative Example 2 | Example 7 | Example 8 |
|---|---|---|---|---|---|---|
| Conditions | Fatty acid methyl ester component | C18 | C18 | C18 | C18 | C18 |
| | Alcohol | DEG | Glycerol | Glycerol | DEG | DEG |
| | Alcohol amount (%) | 0.125 | 0.125 | 0.125 | 0.125 | 0.5 |
| | Catalyst amount (%) | 0.2 | 0.1 | 0.1 | 0.1 | 0.1 |
| | Alkali amount (% relative to catalyst) | 3 | 3 | 4 | 1 | 3 |
| Evaluations | Narrow ratio (%) | 55 | 42 | 60 | 33 | 55 |
| | Induction period (h) | 0 | >2 | >2 | 0 | 0 |
| | Reaction time (h) | 3.5 | >10 | >10 | 3.5 | 5 |
| | Purification time (h) | 5 | 5 | 5 | 5 | 7 |

[Table 3]

|  |  | Example 9 | Example 10 | Comparative Example 3 | Comparative Example 4 | Comparative Example 5 |
|---|---|---|---|---|---|---|
| Conditions | Fatty acid methyl ester component | C18 | C18 | C12 | C12 | C18 |
|  | Alcohol | DEG | DEG | DEG | none | none |
|  | Alcohol amount (%) | 0.125 | 0.125 | 0.125 | 0.125 | 0.125 |
|  | Catalyst amount (%) | 0.3 | 0.05 | 0.1 | 0.1 | 0.1 |
|  | Alkali amount (% relative to catalyst) | 3 | 3 | 3 | 3 | 3 |
| Evaluations | Narrow ratio (%) | 55 | 55 | 55 | 50 | 50 |
|  | Induction period (h) | 0 | 0 | 2 | 0.5 | 0.5 |
|  | Reaction time (h) | 3 | 8 | 4.5 | 6 | 6 |
|  | Purification time (h) | 7 | 4.5 | 5 | 5 | 7 |

Based on comparison of Example 1 and Comparative Examples 3 to 5, it is evident that when the fatty acid methyl ester component contained the C18 to C22 fatty acid methyl ester as the main component, the alcohol described above enabled the induction period to be eliminated, whereas when the fatty acid methyl ester component contained the C12 fatty acid methyl ester as the main component, the above alcohol actually lengthened the induction period.

Based on a comparison of Comparative Examples 1 and 5, it is evident that glycerol actually lengthened the induction period.

Based on a comparison of Examples 1 to 3 and 8 and Comparative Example 5, it is evident that the alcohol described above shortens the reaction time but lengthens the purification time.

Based on a comparison of Examples 1, 4, 5 and 7 and Comparative Examples 1, 2 and 5, it is evident that, compared with the case where glycerol is used, using the alcohol described above enables a high narrow ratio to be obtained with a small alkali amount, without lowering the reaction rate.

Based on a comparison of Examples 1, 6, 9 and 10, it is evident that the catalyst amount has no effect on the induction period, and that if the catalyst amount is increased, the reaction rate increases but the purification time lengthens.

INDUSTRIAL APPLICABILITY

The present invention is able to provide a method for producing a fatty acid polyoxyethylene methyl ether which enables a reduction in the induction period when adding ethylene oxide to a fatty acid methyl ester in the presence of a composite metal oxide catalyst.

**Claims**

1.  A method for producing a fatty acid polyoxyethylene methyl ether, the method comprising adding ethylene oxide to a fatty acid methyl ester component comprising, as a main component, a fatty acid methyl ester having a fatty acid residue of 18 to 22 carbon atoms, wherein the addition is conducted in presence of a composite metal oxide catalyst and at least one alcohol selected from the group consisting of diethylene glycol, ethylene glycol, propylene glycol, ethanol, methanol and isopropyl alcohol.

2.  The method for producing a fatty acid polyoxyethylene methyl ether according to Claim 1, wherein at least a portion of the fatty acid methyl ester having a fatty acid residue of 18 to 22 carbon atoms has an unsaturated fatty acid residue.

3.  The method for producing a fatty acid polyoxyethylene methyl ether according to Claim 1 or 2, wherein the fatty acid methyl ester having a fatty acid residue of 18 to 22 carbon atoms comprises a fatty acid methyl ester having a fatty acid residue of 18 carbon atoms.

4. The method for producing a fatty acid polyoxyethylene methyl ether according to any one of Claims 1 to 3, wherein the fatty acid methyl ester component is a fatty acid methyl ester mixture derived from a distillation fraction of 18 carbon atoms from palm oil, palm kernel oil, coconut oil or soybean oil, a fatty acid methyl ester mixture derived from a distillation fraction of 18 to 22 carbon atoms from rapeseed oil, or a mixture of two or more such mixtures.

## INTERNATIONAL SEARCH REPORT

| | International application No. |
|---|---|
| | PCT/JP2018/047012 |

**A. CLASSIFICATION OF SUBJECT MATTER**
Int.Cl. C07C67/29(2006.01)i, B01J21/10(2006.01)i, C07C69/24(2006.01)i,
        C07C69/58(2006.01)i, C07B61/00(2006.01)n

According to International Patent Classification (IPC) or to both national classification and IPC

**B. FIELDS SEARCHED**

Minimum documentation searched (classification system followed by classification symbols)
Int.Cl. C07C67/29, B01J21/10, C07C69/24, C07C69/58, C07B61/00

Documentation searched other than minimum documentation to the extent that such documents are included in the fields searched
| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2007/113985 A1 (LION CORP.) 11 October 2007, claims, paragraphs [0009], [0021], [0024], [0052]–[0076] & KR 10-2008-0106565 A | 1–4 |
| X | WO 2008/078768 A1 (LION CORP.) 03 July 2008, claims 1-7, paragraphs [0013], [0015], [0020]–[0024] & MY 159968 A | 1–4 |

☐ Further documents are listed in the continuation of Box C.    ☐ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 15 March 2019 (15.03.2019) | 26 March 2019 (26.03.2019) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017250992 A
- WO 2007113985 A
- JP HEI615038 B
- JP HEI7227504 B
- JP HEI6198169 B
- JP HEI6182206 B

- JP HEI5170688 B
- JP HEI271841 B
- JP HEI8268919 B
- JP HEI8169860 B
- JP HEI8169861 B